Europäisches Patentamt

(19) European Patent Office

Office européen des brevets

(11) Publication number: **0 044 532**
**B1**

(12) **EUROPEAN PATENT SPECIFICATION**

(45) Date of publication of patent specification: **23.04.86**

(21) Application number: **81105592.0**

(22) Date of filing: **16.07.81**

(51) Int. Cl.[4]: **C 12 Q 1/00,** C 12 Q 1/50,
C 12 N 9/96

(54) Monothioglycerol as thiol-protector in lyophilized materials.

(30) Priority: **18.07.80 US 170060**

(43) Date of publication of application:
**27.01.82 Bulletin 82/04**

(45) Publication of the grant of the patent:
**23.04.86 Bulletin 86/17**

(84) Designated Contracting States:
**BE CH DE FR IT LI NL**

(56) References cited:
**EP-A-0 019 939**
**US-A-3 448 009**
**US-A-3 627 683**
**US-A-4 118 279**

**CLINICAL CHEMISTRY, vol. 26, July 1980, no. 8
Winston-Salem (N.C.) D.A. NEALON et al.:
"Effect of Serum pH on Storage Stability and
Reaction Lag Phase of Human Creatine Kinase
Isoenzymes", pages 1165-1169**

(73) Proprietor: **E.I. DU PONT DE NEMOURS AND
COMPANY
1007 Market Street
Wilmington Delaware 19898 (US)**

(72) Inventor: **Briggs, Anglis R.
158 Woodshade Drive
Newark, Delaware 19702 (US)**

(74) Representative: **Abitz, Walter, Dr.-Ing. et al
Abitz, Morf, Gritschneder P.O. Box 86 01 09
D-8000 München 86 (DE)**

(56) References cited:
**CHEMICAL ABSTRACTS, vol. 89, no. 25 18th
December 1978, page 379, column 1, abstract
212759s Columbus, Ohio, US G. SZASZ et al.:
"Creatine kinase in serum 5. Effect of thiols on
isoenzyme activity during storage at various
temperatures"**

**CLINICA CHIMICA ACTA, vol. 58, 16th January,
1975, Amsterdam, NI D.S. MIYADA et al.:
"Creatine Kinase Reactivation by thiol
compounds", pages 97-99**

**Description**

Technical field

This invention relates to thiol-protector agents used with sulfhydryl-requiring enzymes and more particularly to monothioglycerol as a thiol-protector in lyophilized materials.

Background art

Certain proteins require the presence of sulfhydryl groups in their structure for activity and stability. In the presence of oxidizing agents, however, including atmospheric oxygen, the sulfhydryl groups are oxidized to disulfide linkages with concomitant deleterious changes in the protein's properties. These problems are magnified when the protein is in serum-based material or lyophilized.

Addition of excess thiols has been utilized as a means of protection for the sulfhydryl groups of proteins. A large number of thiols has been evaluated for effectiveness as a protective agent against oxidation (G. Szasz, "Proceedings of the Second International Symposium on Clinical Enzymology", 1975, pages III-1 to 36). Szasz examined twenty-seven thiol compounds with creatine kinase, an unstable enzyme. Both possible dimercapto (1,3 and 1,2-dimercapto) glycerols were tested for their effectiveness but monothioglycerol [1-mercapto (or 1 - sulfhydryl) - 2,3 - propanediol] was not included in the evaluation.

Monothioglycerol (MTG, 1-sulfhydryl-2,3-propanediol) has been found to be effective for this purpose; its half-life in solutions in equilibrium with atmospheric oxygen is sufficiently long for practical purposes. However, it has been stated by L. G. Morin, in Clinical Chemistry, Volume 23, No. 9, 1569 (1977), at page 1574 that "(monothioglycerol) would undoubtedly not lyophilize". For this reason, MTG has not been utilized as a sulfhydryl-protector in lyophilized materials.

G. Szasz, et al. [Clinical Chemistry, Volume 24, No. 9, 1557 (1978)] also investigated MTG but observed gelling of the serum proteins, with 50 mM MTG per liter, after six hours storage at 37°C. These investigators then selected N-acetyl cystein as the protecting agent of choice for creatine kinase isoenzymes.

Disclosure of the invention

The lyophilized product of this invention consists essentially of:

(A) monothioglycerol, present in the pre-lyophilized solution to an extent of not less than approximately 2 mmoles/liter of solution and in an amount not leading to gelation of the solution;

(B) sulfhydryl-requiring enzyme; and

(C) substantially completely delipified animal serum having low residual levels of enzyme activity.

This product can be reconstituted without showing any significant adverse temperature of hydration effect affording a product in which the enzyme activity remains substantially constant for 24 hours at 4°C.

The invention is further concerned with a method for preparing a lyophilized composition containing sulfhydryl-requiring enzymes, which includes the step of adding thiol-protectors prior to lyophilization, which is characterized in that monothioglycerol is added as the thiol-protector.

Description of the invention

Certain enzymes are "sulfhydryl-requiring", that is, they need to maintain their —SH groups in their free, unoxidized form. Atmospheric oxygen causes the oxidation of the —SH groups to the disulfide (S—S) linkage, thereby diminishing enzyme stability and catalytic activity. Among "sulfhydryl-requiring" enzymes are yeast alcohol dehydrogenase, glyceraldehyde - 3 - phosphate dehydrogenase, papain, RNA polymerase, acetate thiokinase, and creatine kinase isoenzymes.

In contrast, some proteins require disulfide linkages for their activity. Such proteins, for example, ribonuclease, IgG, insulin, do not require thiol-protectors during storage and exposure to atmospheric oxygen.

Many thiols have been utilized previously as thiol-protectors for "SH-requiring" enzymes. Among the best of the thiols for this purpose are N-acetyl cystein (NAC), glutathione (GSH), mercaptoethanol, dithiothreitol, dithioerythritol, and monothioglycerol (MTG). Some of these, such as NAC and GSH, have also been incorporated into enzyme solutions and subsequently lyophilized. In the aforementioned review article, comparing some twenty-seven thiols as thiol-protectors, NAC was found to have optimum overall properties. MTG, however, which has many advantages over other thiols, such as being nonionic and having longer half-life than NAC in enzyme solutions, has been said to be unsuitable for incorporation into enzyme solutions which will be lyophilized.

It has now been found unexpectedly that lyophilized compositions can be prepared by inclusion of MTG into solutions containing "SH-requiring" enzymes and a specially prepared human serum matrix prior to lyophilization. When reconstituted, such products are stable for periods of time sufficient for use in the clinical laboratory. In the lyophilized form, they appear to be stable indefinitely. 2—100 mmoles/liter of MTG in the pre-lyophilized composition is the useful range of concentration with 20 mmoles/liter being a preferred amount. The upper limit is that amount of MTG which does not cause gelation of the composition.

From accelerated experiments it is predicted that when the "SH-requiring" enzymes is CKMM, then products containing this isoenzyme, MTG, and a specially prepared serum, which is substantially completely delipified and has low residual levels of enzyme activity, would exhibit little or no detectable

2

**0 044 532**

loss of enzyme activity in the lyophilized form after one year of storage at 4±2°C. For CKMM, the expected range of concentration in the pre-lyophilized composition is 20—40 enzyme units/liter at the low end of the scale up to approximately 3,000 units/liter at the high end.

The finding that MTG can be utilized with enzymes to afford lyophilized product led to unexpected benefits. To be useful as control products in the clinical analysis field, an enzyme-containing lyophilized product must have two characteristics often very difficult to achieve simultaneously. First, there must be substantially no or only very small temperature of hydration effect and, second, there must only be very slight changes in enzyme activity upon standing after reconstitution (rehydration) of the lyophilized product.

The temperature of hydration effect is the phenomenon of variability of recovered enzyme activity from a lyophilized material upon rehydration caused by the differences in the temperature of water used. For obvious practical purposes, for use in the clinical laboratory, lyophilized products must have a small temperature of hydration effect to permit reproducible analytical results. This temperature of hydration effect is calculated by reconstituting the lyophilized product with water at 20, 25, and 30°C, followed by the measurement of enzyme activity of the rehydrated solutions. The slope of the line fitting these data is determined by a least squares calculation to obtain the temperature of hydration effect in enzyme units/°C. The %/°C value is calculated from the enzyme activity value of 25°C. It is desired that the temperature of hydration effect not exceed ±0.5%/°C. It is further desired that this low temperature of hydration effect be coupled to an enzyme activity change not exceeding ±5% during 24 hours standing at 4°C (after rehydration of the lyophilized material).

In a comparison study, the thiol-protectors N-acetyl cystein (NAC) and the subject of this invention, monothioglycerol (MTG), were incorporated at various levels into a specially prepared human serum matrix containing creatine kinase MM isoenzyme and various levels of a buffer, 2 - amino - 2 - hydroxymethyl - 1,3 - propanediol (TRIS).

The specially prepared substantially completely delipified animal (including human) serum having low residual levels of enzyme activity and its preparation have been described in EP—A1—0019939. This serum comprises the bulk of the pre-lyophilized composition from which the lyophilized product of this invention is prepared.

In this comparison study, the pH of the solutions was adjusted to 7.2 with HCl, the solutions were lyophilized and then rehydrated. Temperature of hydration effect data and those showing the change in CKMM activity upon standing for 24 hr at 4°C are shown below:

### NAC as thiol protector

| Run No. | NAC (mmoles/ liter) | Buffer (TRIS, mmoles/ liter) | Temperature of hydration effect[1] (%/°C) | Change in CKMM activity[1] (%) |
|---|---|---|---|---|
| 1 | 10 | 100 | 0.11 | −10.5 |
| 2 | 10 | 200 | 1.5 | −7.7 |
| 3 | 10 | 300 | 4.1 | +1.8 |
| 4 | 20 | 100 | 0.25 | −6.5 |
| 5 | 20 | 200 | 1.2 | −6.5 |
| 6 | 20 | 300 | 3.5 | +1.5 |
| 7 | 30 | 100 | 0.11 | 10.9 |
| 8 | 30 | 200 | 0.97 | −4.6 |
| 9 | 30 | 300 | 3.5 | +1.1 |

[1]CKMM activity was measured by using CK test packs with E. I. du Pont de Nemours and Company's *aca* instrument.

3

**0 044 532**

MTG as thiol protector

| Run No. | MTG (mmoles/ liter) | Buffer (TRIS, mmoles/ liter) | Temperature of hydration effect[1] (%/°C) | Change in CKMM activity[1] (%) |
|---|---|---|---|---|
| 1 | 10 | 100 | 0.59 | +2.8 |
| 2 | 10 | 200 | 2.4 | +4.1 |
| 3 | 10 | 300 | 3.5 | +7.7 |
| 4 | 20 | 100 | 0.22 | +2.1 |
| 5 | 20 | 200 | 1.6 | +3.5 |
| 6 | 20 | 300 | 3.2 | +6.8 |
| 7 | 30 | 100 | 0.56 | +1.1 |
| 8 | 30 | 200 | 1.1 | +1.8 |
| 9 | 30 | 300 | 4.2 | +8.2 |

[1]See footnote above.

As can be seen from the above tables, while NAC does provide either acceptable temperature of hydration effect or within-limits change of enzyme activity, there were no runs which showed acceptable values for both properties simultaneously. On the other hand, the composition of this invention, utilizing MTG as the thiol protector, is acceptable from both standpoints, especially when low levels of buffer are utilized. (Higher levels of buffer presumably lead to higher levels of ion concentration which may cause a higher temperature of hydration effect).

It was found further that the half-life of the SH-group of NAC in the specially prepared substantially completely delipified human serum described above is approximately 4 hours when measured in a reconstituted TRIS-buffered system (pH=7.2) at 16—20°C. The comparable SH-group half-life for MTG in the same system is 22.6 hours, further indicating the superiority of this thiol protector. The data from which these half-life values were derived are presented below:

N-Acetyl cystein (NAC)
(nominal SH-group concentration prior to lyophilization: 5 mmoles/liter)

| Elapsed time (hr.) | Measured SH-group concentration[1,2] |
|---|---|
| 0 | 2.87 |
| 0.5 | 2.67±0.08 |
| 1 | 2.38±0.20 |
| 2 | 1.92±0.15 |
| 7 | 0.98±0.14 |

[1]SH-concentration is measured by using Ellman's Reagent, 5,5'-dithiobis (2-nitrobenzoic acid)
[2]The calculated constant for the rate of decay is $k=0.1710 \ hr^{-1}$

4

# 0 044 532

Monothioglycerol (MTG)
(nominal SH-group concentration, prior to lyophilization: 20 mmoles/liter)

| Elapsed time (hr.) | Measured SH-group concentration[1,2] |
|---|---|
| 0 | 17.88±2.0 |
| 3.3 | 16.16±0.05 |
| 20 | 10.05±0.07 |

[1]See footnote above
[2]$k_{decay}$=0.030649 hr$^{-1}$

A further proof of the unexpected possibility to prepare a lyophilized product according to the invention was derived from the analysis of the moisture content of the lyophilized product of this invention. Karl Fisher moisture assays carried out on the lyophilized cake-like material, see Example, indicated an average moisture content of 0.696±0.075% by weight, which value is in the normal range for lyophilized products in general. Coupled with the data presented above (approximately 90% of the SH-groups are still present upon reconstitution after lyophilization, t=0), MTG is shown to be a useful thiol-protector possessing all of the required characteristics and unexpectedly affording lyophilized products of superior properties.

The lyophilized product of this invention can be reconstituted with water to obtain products useful as a calibrator for use in the clinical laboratory. Since the lyophilized product is stable, it can be rehydrated at the appropriate time in the clinical laboratory and used as a control product in conjunction with, for example, automatic clinical analyzers utilized in measuring enzyme activity such as creatine kinase.

Example
A. Preparation of pre-lyophilized product
To a 2-liter quantity of human serum prepared according to EP—A1—0019939 is added 40 mmoles of MTG at room temperature. To this solution is then added a CKMM solution (itself having an MTG concentration of 20 mmoles/liter), to obtain a final enzyme activity of 816 units/liter. This solution is then diluted with an equal volume of water. An assay of this final solution shows an enzyme activity of 426 units/liter.

B. Preparation of lyophilized product
The diluted solution from (A) above is pumped into amber vials, 6.2 ml/vial, the vials are placed onto the shelves of a lyophilizer, and the temperature of the shelves is lowered to −20°C. The contents of the vials are thus frozen placed under a 40—80 micron vacuum, and maintained at −20°C for 12 hours. After this time period the shelf temperature is allowed to rise to +10°C and the vials are kept at this temperature, still under vacuum, for 24 hours. The contents of the vials are finally dried under vacuum for 2 hours at 25°C, resulting in a dry cake weighing approximately 197 mg. The vials are sealed under a 40-micron vacuum and stored at 4±2°C.
The lyophilized product can be tested by rehydrating the contents of a vial with 3 ml of deionized water and analyzed for enzyme activity change and temperature of hydration effect.

Two pilot lots prepared as described above show temperature of hydration effect of 0.12%/°C and 0.21%/°C, respectively, well within the desired limit of 0.5%/°C and +0.8% and +0.85%, respectively, of activity change upon standing. The average freeze-dried product in a vial contains 2.4 enzyme units of CKMM/3 ml of reconstituted solution.

**Claims**

1. A lyophilized product of a solution consisting essentially of:
(A) monothioglycerol, present in the pre-lyophilized solution to an extent of not less than 2 mmoles/liter of solution and in an amount not leading to gelation of the solution;
(B) sulfhydryl-requiring enzyme; and
(C) substantially completely delipified animal serum having low residual levels of enzyme activity.
2. The lyophilized product of Claim 1 wherein the monothioglycerol is present to an extent of 2—100 mmoles/liter of solution.
3. The lyophilized product of Claim 1 wherein the monothioglycerol is present at a concentration of 20 mmoles/liter of solution.
4. The lyophilized product of Claim 1 wherein the sulfhydryl-requiring enzyme is CKMM.
5. A rehydrated composition of a lyophilized product consisting essentially of an aqueous solution of:

5

(A) monothioglycerol, present in the pre-lyophilized solution to an extent of not less than 2 mmoles/liter of solution and in an amount not leading to gelation of the solution;

(B) sulfhydryl-requiring enzyme; and

(C) substantially completely delipified animal serum having low residual levels of enzyme activity. wherein the composition shows no significant temperature of hydration effect and whose enzyme activity remains substantially constant for 24 hours at 4°C.

6. The rehydrated composition of Claim 5 wherein the temperature of hydration effect is less than ±0.5%/°C and the enzyme activity changes less than ±5%.

7. Method for preparing a lyophilized composition containing sulfhydryl-requiring enzymes which includes the step of adding thiol-protectors prior to lyophilization, characterized in that monothioglycerol is added as the thiol-protector.

8. Method of Claim 7 characterized in that the amount of monothioglycerol added to the composition to be lyophilized is 2—100 mmoles/liter of the composition.

**Patentansprüche**

1. Ein lyophilisiertes Produkt einer Lösung, bestehend im wesentlichen aus:

A) Monothioglyzerin, vorhanden in der vorlyophilisierten Lösung in einem Ausmaß von nicht weniger als 2 mMol/Liter Lösung und in einer Menge die nicht zur Gelbildung der Lösung führt;

B) Sulfhydryl-erforderndes Enzym; und

C) im wesentlichen vollständig delipifiziertes tierisches Serum mit niedrigen Restgehalten an Enzymaktivität.

2. Das lyophilisierte Produkt nach Anspruch 1, worin das Monothioglyzerin in einem Ausmaß von 2—100 mMol/Liter Lösung vorhanden ist.

3. Das lyophilisierte Produkt nach Anspruch 1, worin das Monothioglyzerin in einer Konzentration von 20 mMol/Liter Lösung vorhanden ist.

4. Das lyophilisierte Produkt nach Anspruch 1, worin das Sulfhydryl-erfordernde Enzym CKMM ist.

5. Ein rehydratisierte Zusammensetzung eines lyophilisierten Produkts, bestehend im wesentlichen aus einer wäßrigen Lösung von:

A) Monothioglyzerin, vorhanden in der vorlyophilisierten Lösung in einem Ausmaß von nicht weniger als 2 mMol/Liter Lösung und in einer Menge, die nicht zur Gelbildung der Lösung führt;

B) Sulfhydryl-erforderndes Enzym; und

C) im wesentlichen vollständig delipiziertes tierisches Serum mit niedrigen Restgehalten an Enzymaktivität,

worin die Zusammensetzung keine wesentliche Hydratations-Effekt-Temperatur zeigt und deren Enzymaktivität im wesentlichen während 24 Stunden bei 4°C konstant bleibt.

6. Die rehydratisierte Zusammensetzung nach Anspruch 5, worin die Hydratations-Effekt-Temperatur weniger als ±0,5%/°C ist und sich die Enzymaktivität weniger als ±5% ändert.

7. Verfahren zur Herstellung einer lyophilisierten Zusammensetzung, die Sulfhydryl-erfordernde Enzyme enthält, das die Stufe das Zusatzes von Thiol-Protektoren vor der Lyophilisierung umfaßt, dadurch gekennzeichnet, daß als Thiol-Protektor Monothioglyzerin zugesetzt wird.

8. Verfahren nach Anspruch 7, dadurch gekennzeichnet, daß die Menge des zu der zu lyophilisierenden Zusammensetzung zugesetzten Monothioglyzerins 2—100 mMol/Liter der Zusammensetzung beträgt.

**Revendications**

1. Un produit lyophilisé d'une solution constituée essentiellement de:

(A) monothioglycérol, présente dans la solution de pré-lyophilisation en une proportion non inférieure à 2 mmoles/litre de solution et en une quantité qui ne conduit pas à la gélification de la solution;

(B) un enzyme nécessitant le groupe sulfhydryle; et

(C) sérum animal pratiquement complètement délipifié présentant de faibles niveaux résiduels d'activité enzymatique.

2. Le produit lyophilisé de la revendication 1, dans lequel le monothioglycérol est présent en une proportion de 2—100 mmoles/litre de solution.

5. Le produit lyophilisé de la revendication 1, dans lequel le monothioglycérol est présent à une concentration de 20 mmoles/litre de solution.

4. Le produit lyophilisé de la revendication 1, dans lequel l'enzyme nécessitant le groupe sulfhydryle est la CKMM.

5. Une composition réhydratée d'un produit lyophilisé constituée essentiellement d'une solution aqueuse de:

(A) monothioglycérol, présent dans la solution de pré-lyophilisation en une proportion non inférieure à 2 mmoles/litre de solution et en une quantité qui ne conduit pas à la gélification de la solution;

(B) un enzyme nécessitant le groupe sulfhydryle; et

(C) sérum animal pratiquement complètement délipifié présentant de faibles niveaux résiduels d'activité enzymatique,

**0 044 532**

la composition n'étant pas sujette à une influence notable de la température d'hydratation et son activité enzymatique restant sensiblement constante pendant 24 heures à 4°C.

6. La composition réhydratée de la revendication 5, dans laquelle l'influence de la température d'hydratation est inférieure à ±0,5%/°C et les variations d'activités enzymatiques dont inférieures à ±5%.

7. Procédé de préparation d'une composition lyophilisée contenant des enzymes nécessitant le groupe sulfhydryle, qui comprend l'étape consistant à ajouter des protecteurs de thiols avant la lyophilisation, caractérisé en ce que du monothioglycérol est ajouté en tant que protecteur de thiols.

8. Procédé de la revendication 7, caractérisé en ce que la quantité de monothioglycérol ajoutée à la composition à lyophiliser est de 2—100 mmoles/litre de la composition.